(19) **Europäisches Patentamt**
**European Patent Office**
**Office européen des brevets**

(11) **EP 3 784 296 B1**

(12) **FASCICULE DE BREVET EUROPEEN**

(45) Date de publication et mention
de la délivrance du brevet:
**10.04.2024   Bulletin 2024/15**

(21) Numéro de dépôt: **19719295.8**

(22) Date de dépôt: **24.04.2019**

(51) Classification Internationale des Brevets (IPC):
***A61L 2/04*** *(2006.01)*        ***A61Q 19/00*** *(2006.01)*

(52) Classification Coopérative des Brevets (CPC):
**A61L 2/0023; A61K 8/06; A61L 2/04; A61Q 19/00;**
A61K 2800/805; A61L 2202/21

(86) Numéro de dépôt international:
**PCT/EP2019/060547**

(87) Numéro de publication internationale:
**WO 2019/207017 (31.10.2019 Gazette 2019/44)**

(54) **PROCÉDÉ ET DISPOSITIF POUR LA FABRICATION D'UNE ÉMULSION DERMO-COSMÉTIQUE STÉRILE COMPRENANT UN GÉLIFIANT**

VERFAHREN UND VORRICHTUNG ZUR HERSTELLUNG EINER STERILEN DERMOKOSMETISCHEN EMULSION MIT EINEM GELIERMITTEL

METHOD AND DEVICE FOR PRODUCING A STERILE DERMOCOSMETIC EMULSION COMPRISING A GELLING AGENT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité:  **24.04.2018   FR 1870482**
                **24.04.2018   FR 1870483**

(43) Date de publication de la demande:
**03.03.2021   Bulletin 2021/09**

(73) Titulaire: **Pierre Fabre Dermo-Cosmétique
81500 Lavaur (FR)**

(72) Inventeur: **DELAUNAY, Jean-Claude
34800 CLERMONT-L'HERAULT (FR)**

(74) Mandataire: **Ipside
Tour Trinity
1 B Place de la Défense
92400 Courbevoie (FR)**

(56) Documents cités:
**EP-A1- 1 314 361        EP-A1- 1 588 697
WO-A1-2013/007755**

EP 3 784 296 B1

## Description

L'invention concerne un procédé et un dispositif pour la fabrication d'une émulsion dermo-cosmétique stérile comprenant un gélifiant, ledit procédé utilisant une stérilisation par effet de la chaleur de la base émulsive.

## Domaine technique

L'invention est plus particulièrement destinée à la stérilisation d'une préparation dermo-cosmétique sous la forme d'un gel, d'une crème ou d'un baume.

Une telle préparation, lorsque le niveau de stérilité est suffisamment élevé et qu'elle est conditionnée dans un emballage adéquat, présente une longue durée de conservation, sans qu'il ne soit nécessaire d'y ajouter des produits conservateurs, que des esters de l'acide para-hydroxybenzoïque, des sorbates ou des esters de glycol.

Ces adjuvants ne participent pas aux principes actifs visés pour les préparations qui les contiennent, mais peuvent cependant être mal tolérés.

Il est par conséquent souhaitable de proposer des produits, notamment dans le domaine dermo-cosmétique, n'utilisant pas de tels adjuvants conservateurs, mais qui présentent une durée de conservation et une propreté microbienne adéquates.

Ainsi, les formulations visées par le procédé objet de l'invention sont dépourvues de conservateurs susceptibles d'être responsables d'intolérances cutanées, notamment de tout ammonium quaternaire, éthanol, phénols, amidines, dérivés d'isothiazolone, esters parahydroxybenzoïques (connus sous le nom de parabens), etc.

Le terme conservateur désigne ainsi toute substance apte à empêcher le développement de micro-organismes dans la composition par son action antimicrobienne propre.

Le terme conservateur, au sens de la présente invention, englobe aussi bien les conservateurs *stricto sensu,* notamment tels que réglementairement listés (par exemple dans le Règlement CE n° 1223/2009 du Parlement Européen et du Conseil du 30 novembre 2009 relatif aux produits cosmétiques, définition article 2.1.l et Annexe V), que les substances dites « *preservative-like »,* non listées par la réglementation, mais présentant tout de même une fonction de conservateur.

De telles substances sont susceptibles d'être présentes dans les formules cosmétiques pour des fonctions autres, telles que parfumer, tonifier, raffermir, etc., alors qu'elles présentent également des propriétés antimicrobiennes, qui empêchent ainsi la croissance des micro-organismes.

Les formulations des produits visés par le procédé objet de l'invention ne comprennent ni conservateurs listés dans l'Annexe V du Règlement CE n° 1223/2009 du Parlement Européen et du Conseil du 30 novembre 2009 relatif aux produits cosmétiques, ni conservateur non listé ou « *preservative-like ».*

Ainsi, sont par exemple exclus de la formulation des produits visés par le procédé objet de l'invention, et compte tenu de leur effet de conservateur, les composés tels que le caprylyl glycol, le pentylèneglycol, l'éthylhexylglycérine et plus généralement tout adjuvant à effet bactéricide, fongicide, bactériostatique ou fongistatique, notamment des parfums sur base alcoolique et des huiles essentielles présentant ces propriétés.

Les produits visés par l'invention, comprennent une base émulsive comprenant une phase grasse constituant la phase interne ou dispersée de l'émulsion et une phase aqueuse constituant la phase externe ou continue de l'émulsion. Cette base émulsive constitue plus de 90 % du volume du produit, le plus souvent plus de 99 % du volume du produit.

Ces produits comprenent en outre des principes actifs, lesquels principes actifs, sont dissous, liés ou en suspension dans la phase grasse ou la phase aqueuse, et un gélifiant.

Le gélifiant agit comme un agent viscosant et stabilisant de l'émulsion, et les produits finaux visés par l'invention ont une viscosité dynamique à température ambiante généralement comprise entre 5 000 cP et 25 000 cP sans que cette fourchette ne soit limitative.

La viscosité d'un produit dermo-cosmétique et la stabilité dans le temps de cette viscosité sont des propriétés organoleptiques importantes du produit, la viscosité déterminant la facilité et le plaisir d'application du produit selon son indication cosmétique.

Les propriétés organoleptiques du produit et son efficacité cosmétique sont également liées à la qualité de l'émulsion, une émulsion fine présentant des micelles de petite dimension et réparties de manière homogène dans le produit est plus agréable lors de l'application topique, mais également pus efficace sur les couches supérieures de l'épiderme. Les propriétés organoleptiques de l'émulsion sont également plus stables lorsque l'émulsion est fine.

Dans un produit exempt de tout type de conservateur, tels que ceux visés par l'invention, la conservation du produit n'est obtenue qu'en stérilisant celui-ci à un haut degré de stérilité, comparable, voire supérieur, à celui utilisé pour des produits pharmaceutiques injectables.

Les produits dermo-cosmétiques visés par l'invention doivent être produits en très grande quantité avec ce degré de stérilité élevé, la demande actuelle pour ce type de produit étant très élevée avec en moyenne une vente toutes les 2 secondes dans le monde en 2018.

La stérilité est définie par la norme EN 556 et la pharmacopée européenne en vigueur, comme la probabilité d'un micro-organisme de proliférer dans ledit produit. Typiquement, ladite probabilité, pour un produit dit stérile, est inférieure à $10^{-6}$.

Ce niveau de stérilité correspond à un indice de stérilité F0 égal à 15. Cette valeur F0, dont la méthode de détermination est définie par la pharmacopée européenne en vigueur, donne un temps, exprimé en minutes, quantifiant l'effet létal de la chaleur humide à 121°C sur les micro-organismes viables.

L'effet létal est mesuré par rapport à un germe de référence, le géobacillus stéarothermophilus sporulé, ces bactéries étant particulièrement résistantes et tolérantes à la chaleur.

Ainsi, l'effet de stérilisation minimum visé est celui qu'aurait produit sur ces germes, dans le milieu étudié, un traitement thermique de 15 minutes à 121°C. Ce traitement croit en temps de manière exponentielle avec la baisse de la température en fonction de la nature du micro-organisme dont la destruction est visée par le traitement. La valeur de F0 est ainsi donnée par la formule :

$$F0 = t.10^{(T-121/z)}$$

où :

- t est le temps de traitement exprimé en minutes ;
- z a la dimension d'une température et est défini par la résistance thermique du micro-organisme considéré. La valeur de z est définie expérimentalement en regard d'un paramètre D.
- D est un temps de réduction décimal et mesure le temps, à une température donnée, ici à 121°C, pour réduire la concentration du micro-organisme considéré de 90 %. Pour géobacillus stéarothermophilus, D est égal à une minute.
- T est la température du traitement, exprimée en °C dans cette formule.

Ainsi, z est la variation de température qui modifie la valeur de D d'un facteur 10, pour géobacillus stéarothermophilus, z est égal à 10 °C, ces facteurs D et z sont fonction du milieu et varient notamment selon le type d'émulsion.

Ainsi la valeur de F0 est liée à la probabilité d'avoir un micro-organisme susceptible de proliférer dans le produit, ou plus précisément à l'abattement du nombre de micro-organismes contenus dans le produit avant et après l'opération de stérilisation.

Plus la température de traitement *T* et le temps de maintien t sont élevés et plus cette probabilité est faible, l'abattement étant d'autant plus élevé.

Ainsi, dans un produit non stérile comprenant $10^9$ germes par unité de volume, un traitement correspondant à un indice F0 de 15, produit un abattement de 15 Log du nombre de germes pour la même unité de volume, ramenant celui-ci à $10^{-6}$.

Ce niveau de stérilisation élevé permet une longue conservation du produit dès lors que celui-ci est conditionné dans un contenant évitant toute rétro-contamination, y compris pour des conditionnements de volume important, jusqu'à 400 ml.

Une stérilisation à 141°C et 10 secondes de maintien produit un F0 de 15, tout comme une stérilisation à 145°C et un temps de maintien de 4 secondes. Une stérilisation à 145°C pour un temps de maintien de 6 secondes produit un F0 de 22.

Lorsque la stérilisation n'est pas réalisée par l'action létale de la chaleur sur les micro-organismes, par exemple lorsque celle-ci est réalisée par ultrafiltration, par l'exposition à un faisceau d'électrons, à une lumière pulsée ou à une pression extrême, un F0 équivalent est déterminé par l'abattement du nombre de germes par unité de volume sous l'effet du procédé. Ainsi, tout procédé de stérilisation peut être caractérisée par une valeur F0 définissant l'abattement du nombre de micro-organismes qu'il réalise dans le produit traité.

L'invention s'adresse à un procédé de stérilisation continue d'un produit, dans lequel, un lot de produit (pouvant atteindre plusieurs tonnes) est stérilisé avant d'être conditionné selon un débit atteignant plusieurs dizaines de litres à la minutes, à la différence des procédés de stérilisation individuels, où le produit est stérilisé dans son conditionnement ou, où une dose réduite de produit est stérilisée juste avant son conditionnement.

Parmi les procédés de stérilisation connus, les procédés mettant en oeuvre un traitement thermique du produit, et qui sont dérivés du domaine agroalimentaire, sont ceux qui offrent généralement la productivité la plus élevée.

Toutefois, dans leur application au domaine agroalimentaire, ces procédés visent des niveaux de stérilité, généralement un F0 de l'ordre de 3 à 7, nettement inférieurs au niveau de stérilité visé pour la conservation des produits dermo-cosmétiques sans aucun conservateur.

L'atteinte d'un niveau de stérilité élevé selon ces procédés nécessite de soumettre le produit traité à des sollicitations themomécaniques sévères.

Pour certains produits ces sollicitations thermomécaniques conduisent à une dégradation significative de leurs propriétés organoleptiques, tels que l'aspect, l'homogénéité, l'onctuosité, ou la texture du produit ainsi traité, sauf à prendre des précautions particulières.

Ainsi, la mise en oeuvre de ces procédés de stérilisation à forte productivité dans le domaine des produits dermo-cosmétique est d'autant plus délicate que les produits à stériliser sont visqueux, notamment pour les crèmes épaisses ou les baumes et plus généralement pour les produits présentant une viscosité supérieure à 10 000 cP.

## Technique antérieure

Le document WO 2013/007755 décrit un procédé permettant de stériliser un produit dermo-cosmétique sous la forme d'une émulsion jusqu'à un niveau de stérilité élevé, c'est-à-dire un F0 de 22, par un traitement thermique consistant en un chauffage direct de l'émulsion par infusion de vapeur.

Pour éviter de dégrader le produit, ce procédé utilise des conditions spécifiques de préchauffage et de

refroidissement du produit avant et après la stérilisation par infusion.

Si ce procédé donne globalement satisfaction, il est moins adapté au traitement d'un produit cosmétique de viscosité relativement élevée et comprenant un gélifiant, dans le sens où la viscosité encore élevée du produit lors de son passage dans l'installation limite le débit de traitement atteignable par ce procédé qui est un procédé de traitement en continu.

En effet, dans un procédé de traitement continu, le débit de produit est constant tout le long du procédé, assuré par des moyens de pompage du produit répartis dans l'installation. La puissance des moyens de pompage installés dépend des pertes de charges dynamiques que subi le produit en traversant l'installation, lesquelles, pour une installation donnée, dépendent de la viscosité dynamique du produit : plus cette viscosité est élevée et plus les pertes de charges sont élevées. Par ailleurs, la stabilité du produit limite également la puissance des pompes installée, une pression de pompage trop importante pouvant également dégrader le produit.

Ainsi, les sollicitations thermomécaniques subies par le produit au cours du cycle de stérilisation par infusion tendent à réduire sa viscosité finale, cet effet étant particulièrement marqué pour les produits très visqueux tels que les baumes, de sorte qu'une chute de la viscosité du produit stérilisé atteignant 40 % par rapport au produit initial est parfois constatée avec ce type de produit visqueux, du fait de la mise en oeuvre du procédé de stérilisation continu.

Le document WO 2007/148022 décrit un procédé de stérilisation à haute température et en continu d'un produit dermo-cosmétique, cette fois par un chauffage et un refroidissement indirects.

Selon ce document le produit cosmétique à traiter se déplace entre les deux extrémités d'un conduit de petit diamètre, mû par des moyens de pompage sous pression relativement importante, jusqu'à 160 bars, en traversant des bains portés aux températures de traitement. Le procédé comprend un préchauffage du produit, un chauffage à haute température suivi d'un refroidissement jusqu'à température ambiante.

Bien que le chauffage et le refroidissement soient relativement rapides ceux-ci restent moins énergiques que lors d'un chauffage direct tel que dans le procédé par infusion, de sorte que le produit reste globalement plus longtemps à haute température ce qui accroît les risques de dégradation.

De plus, le traitement d'un produit très visqueux selon ce procédé nécessite des pressions de pompage importantes, ce qui sollicite fortement le produit et limite les débits atteignables.

Le document EP 1 588 697 décrit un produit cosmétique sous la forme d'un gel comprenant un gélifiant.

Après l'obtention de l'émulsion comprenant le gélifiant neutralisé, c'est-à-dire un produit très visqueux, celle-ci est stérilisée à une température de 120°C.

Ainsi, pour obtenir un niveau de stérilité F0 de 15, un tel procédé nécessite de maintenir l'émulsion à cette température pendant 19 minutes avec un fort risque de dégradation thermique de l'émulsion.

Par ailleurs la viscosité importante de l'émulsion comprenant le gélifiant à l'état neutralisé rend délicate l'utilisation d'un procédé de stérilisation continu où le débit est conservé d'un bout à l'autre du procédé.

## Problème technique

L'un des problèmes techniques visé par le procédé et les dispositifs objets de l'invention est d'obtenir un produit stérile à la viscosité désirée dans le contexte d'un traitement thermique en continu à haut débit de traitement.

Un haut débit de traitement est un débit supé-rieur à 2 m$^3$/h. Un tel débit est hors d'atteinte des procédés décrits dans les documents WO 2013/007755 et WO 2007/148022 dans le cas d'un produit très visqueux, plus particulièrement dans le cas d'un produit comprenant un gélifiant ou associant un épaississant et un gélifiant.

La conservation de la viscosité du produit à l'issue de la stérilisation est également hors d'atteinte de ces procédés s'agissant d'un produit très visqueux, ceci d'autant plus que le débit est élevé, donc la pression de circulation et le taux de cisaillement auquel est soumis le produit.

## Résumé de l'invention

L'invention vise à remédier à ces inconvénients et concerne à cette fin un procédé selon la présente revendication 1.

Ainsi, le gélifiant est introduit dans la pré-émulsion sous la forme d'un polymère acide, non neutralisé, c'est-à-dire que les chaînes moléculaires du gélifiant sont enroulées et serrées et ne modifient pas ou peu la viscosité de la pré-émulsion.

La neutralisation du gélifiant consiste à ajouter à ladite pré-émulsion une base minérale ou organique, qui favorise la création de liaisons de charges négatives sur les longues chaînes dudit gélifiant et le déroulement desdites chaînes, augmentant ainsi la viscosité du produit et sa stabilité.

La réalisation de cette étape de neutralisation après la stérilisation de la pré-émulsion, permet d'obtenir une pré-émulsion fluide particulièrement résistante au traitement de stérilisation et d'augmenter ainsi le débit de traitement de l'installation.

L'invention est avantageusement mise en oeuvre selon les modes de réalisation et les variantes exposés ci-après, lesquels sont à considérer individuellement ou selon toute combinaison techniquement opérante.

Avantageusement T1 est choisie pour obtenir une viscosité inférieure ou égale à 300 cP de la pré-émulsion à cette température. Ainsi la pré-émulsion est fluide et permet d'atteindre des débits de traitement élevés selon le procédé de traitement continu objet de l'invention.

Selon un exemple de réalisation, T1 est comprise entre 80 °C et 90 °C.

Selon un mode de réalisation du procédé, l'étape ii) comprend les étapes consistant à :

ii.a. porter la pré-émulsion de la température T1 à la température T2 par infusion de vapeur ;

ii.b. maintenir la pré-émulsion à la température T2 pendant un temps t de sorte à obtenir une stérilisation de la pré-émulsion selon un niveau de stérilité F0 égal ou supérieur à 15 ;

ii.c. refroidir la pré-émulsion à une température comprise entre T1 et T1-5°C par en refroidissement flash.

Le procédé de stérilisation par infusion permet de chauffer de manière quasi instantanée la pré-émulsion à une température élevée, typiquement de l'ordre de 145°C. La fluidité de la pré-émulsion à la température T1, rend celle-ci particulièrement adaptée à ce procédé et permet d'obtenir un débit de traitement élevé.

Le refroidissement flash, typiquement associé à l'opération de stérilisation par infusion, permet en outre d'extraire du produit, sous forme de vapeur, l'eau absorbée par celui-ci lors du chauffage par infusion de vapeur.

Avantageusement, selon ce mode de réalisation, T2 est compris entre 141 °C et 145 °C et t est compris entre 10 secondes et 4 secondes.

Selon un autre mode de réalisation, l'étape ii) du procédé objet de l'invention comprend les étapes consistant à :

ii.x. porter la pré-émulsion de la température T1 à la température T2 par un chauffage indirect dans un échangeur de chaleur ;

ii.y. maintenir la pré-émulsion à la température T2 dans l'échangeur de chaleur pendant un temps t pour obtenir un degré de stérilisation F0 égal ou supérieur à 15 ;

ii.z. refroidir la pré-émulsion à une température égale ou inférieure à T1 par un refroidissement indirect dans un échangeur de chaleur.

Ce mode de réalisation assure que la phase aqueuse introduite dans la pé-émulsion à l'étape i) n'est pas dénaturée le long du processus de stérilisation.

Pour ces deux modes de réalisation du procédé, l'étape i) consiste avantageusement à obtenir une pré-émulsion grossière comprenant des tailles et une répartition des micelles non homogènes. Ainsi le temps de malaxage lors de l'étape i) est réduit et la productivité augmentée.

Avantageusement, les étapes ii.x) à ii.z) sont mises en oeuvre au moyen d'échangeurs thermiques à surface raclée.

L'invention concerne également une installation pour la mise en oeuvre du procédé selon son mode de réalisation intégrant une stérilisation par infusion, selon la revendication 8.

L'invention concerne également une installation pour la mise en oeuvre du procédé selon son mode de réalisation par chauffage indirect, selon la revendication 9.

**Brève description des dessins**

L'invention est exposée ci-après selon ses modes de réalisation préférés, nullement limitatifs, et en référence aux figures 1 à 7, dans lesquelles :

- la figure 1 représente des exemples de micrographie d'une émulsion, figure 1A une émulsion grossière et figure 1B une émulsion fine ;
- la figure 2 montre des exemples de répartition et d'homogénéité des micelles dans une émulsion fine et dans une émulsion grossière, telles qu'elles peuvent être obtenue à partir d'une analyse d'image de micrographies semblables à celles de la figure 1, la figure 2A représente un exemple de distribution de la dimension des micelles, la figure 2B montre un exemple de distributions des distances entre les micelles et leurs plus proches voisins ;
- la figure 3 montre des exemples d'évolution de la viscosité d'un produit dermo-cosmétique en fonction de la température, du taux de cisaillement appliqué et de la viscosité initiale du produit ;
- la figure 4 montre l'évolution de la viscosité d'un produit cosmétique en soumis à un cycle de cisaillement à taux croissants puis décroissants et l'influence de la température sur la réponse du produit à ce cycle, figure 4A à température ambiante, figure 4B à 50°C ;
- la figure 5 est un organigramme du procédé objet de l'invention ;
- la figure 6 représente un exemple de réalisation d'une installation de stérilisation mettant en oeuvre le procédé objet de l'invention avec une stérilisation par infusion ;
- et la figure 7 représente un exemple de réalisation d'une installation de stérilisation mettant en oeuvre le procédé objet de l'invention avec une stérilisation par chauffage indirect.

**Description des modes de réalisation**

Selon un exemple de mise en oeuvre, un produit demo-cosmétique visé par l'invention se présente sous la forme d'une émulsion comprenant une phase continue aqueuse et une phase dispersée grasse. L'invention est cependant applicable à un produit comprenant une phase continue grasse et une phase dispersée aqueuse.

Ledit produit, une fois finalisé, est d'une viscosité comprise en 5 000 cP et 25 000 cP. La phase grasse comprend par exemple un ou plusieurs ingrédients choisis parmi les matières grasses d'origine végétale pour leur teneur en acides gras et en triglycérides physiologiques. La phase aqueuse est une eau déminéralisée ou une eau d'origine thermale.

Le produit contient également des principes actifs présents dans la phase grasse ou dans la phase aqueuse, tels qu'un agent hydratant et/ou un agent émollient.

Finalement le produit comprend des agents de texture tels qu'un épaississant et un gélifiant. Ces produis permettent d'ajuster la viscosité du produit et de stabiliser l'émulsion dans le temps vis-à-vis des phénomènes de crémage, relargage, coalescence, floculation etc. qui tendent à détruire l'homogénéité de répartition de la phase grasse dans la phase aqueuse.

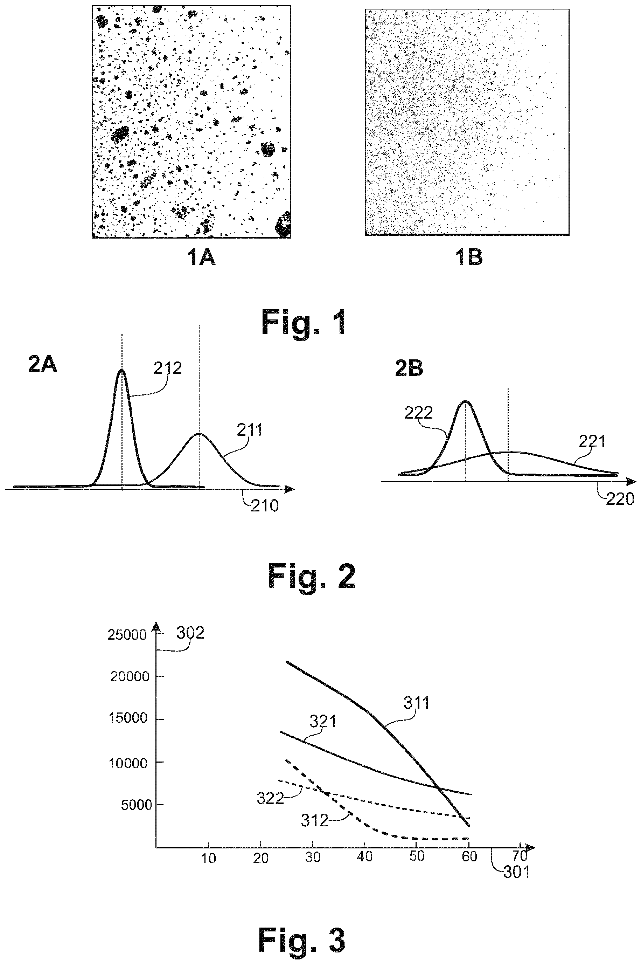

Figure 1, outre ses propriétés macroscopiques, un produit dermo-cosmétique émulsionné est caractérisé par la finesse de l'émulsion, c'est-à-dire par la dimension des micelles de phase grasse et leur répartition plus ou moins homogène dans un volume de produit.

La figure 1A montre un exemple de micrographie d'une émulsion grossière, et la figure 1B un exemple d'une micrographie d'une émulsion fine, les deux micrographies étant à la même échelle. Sur ces images la phase grasse apparaît comme des tâches sombres et la phase aqueuse apparaît en fond blanc.

Figure 1A qualitativement, dans l'émulsion grossière, la phase grasse se répartie selon des micelles de dimensions variables et comportant des micelles de diamètre important, de quelques dizaines de micromètres, et réparties de manière non homogène sur une surface d'analyse donnée.

Figure 1B, dans l'émulsion fine les micelles sont de taille homogène, de petite dimension et réparties de manière homogène sur une surface d'analyse donnée.

Figure 2, ces paramètres sont quantifiables par une analyse d'image en appréciant, par mesure et comptage, figure 2A, la distribution statistique de la taille (210) des micelles sur une surface d'analyse et figure 2B en appréciant par mesure et comptage, la distribution statistique de la distance (220) entre micelles les plus proches sur cette même surface d'analyse.

Les distributions (212, 222) correspondant à l'émulsion fine ont une variance plus faible que les distributions (211, 221) correspondant à l'émulsion grossière. Dans la pratique, les distributions obtenues ne sont pas nécessairement gaussiennes.

L'obtention d'une émulsion fine comprenant des micelles de petite dimension homogènes en taille et bien réparties, participe à l'efficacité du produit dermo-cosmétique, conférant audit produit une texture particulièrement agréable au contact de la peau, moins collante, plus facile et plus rapide à étaler.

Le produit est également plus stable.

La finesse des micelles permet lors d'une application topique, une pénétration plus facile de celles-ci entre les cornéocytes, et un comblement plus rapide et efficace des lamelles lipidiques protectrices de la peau. Le comblement lipidique cornéocytaire en est d'autant plus rémanent.

Figure 3, la viscosité d'un produit dermo-cosmétique émulsionné visé par l'invention évolue avec la température et avec le taux de cisaillement auquel le produit est soumis.

La figure 3 montre un exemple d'évolution de la viscosité dynamique (302), exprimée en cP, avec la température (301), exprimée en °C, pour un baume (311, 312), de viscosité 21 700 cP à température ambiante, et pour une crème (321, 322), de viscosité 13 100 cP à température ambiante, ceci pour un taux de cisaillement de 7,6 s$^{-1}$ (311, 321) et pour un taux de cisaillement de 17,9 s$^{-1}$ (312, 322).

La viscosité dynamique est évaluée, par exemple, au moyen d'un rhéomètre, comprenant un mobile (cylindre ou cône) cisaillant l'émulsion entre la paroi dudit mobile et une paroi fixe.

Ces essais permettent de simuler et de comprendre l'influence des sollicitations thermomécaniques sur la viscosité et la stabilité d'un produit dermo-cosmétique au cours d'un traitement de stérilisation en continu. Ils ne prennent pas en compte l'influence de l'absorption et de relargage de l'eau au cours d'un traitement de stérilisation par infusion et refroidissement flash.

La forme de courbes montre que la viscosité du produit chute avec la température. La comparaison des courbes (311, 312) relatives au produit visqueux, ou au produit moins visqueux (321, 322), montre que la viscosité du produit décroît également lorsque le taux de cisaillement augmente.

La comparaison des courbes relatives au produit visqueux (311, 312) avec celles relatives au produit moins visqueux (321, 322) montre que l'effet de la température ainsi que l'effet du cisaillement produisent une chute de viscosité d'autant plus marquée que le produit est visqueux.

Ainsi, la viscosité chute sous l'effet combiné de la température et du taux de cisaillement, l'effet est d'autant plus marqué que le produit initial est plus visqueux.

Finalement la comparaison de l'écart relatif de viscosité entre les courbes (311, 321) obtenues pour un faible taux de cisaillement et celles (312, 322) montre que l'influence du cisaillement est également plus marquée sur le produit le plus visqueux.

Pour ces raisons, si un produit dermo-cosmétique fluide tel qu'un lait d'une viscosité inférieure à 2 000 cP est apte à subir un traitement de stérilisation même sévère sans influence sur sa viscosité finale, il n'en est pas de même pour un produit visqueux tel qu'une crème épaisse ou un baume.

Dans certaines conditions la chute de la viscosité du produit observée avec l'augmentation de la température et de la sollicitation mécanique témoigne de la dégradation des qualités organoleptiques du produit, notamment de la casse de l'émulsion.

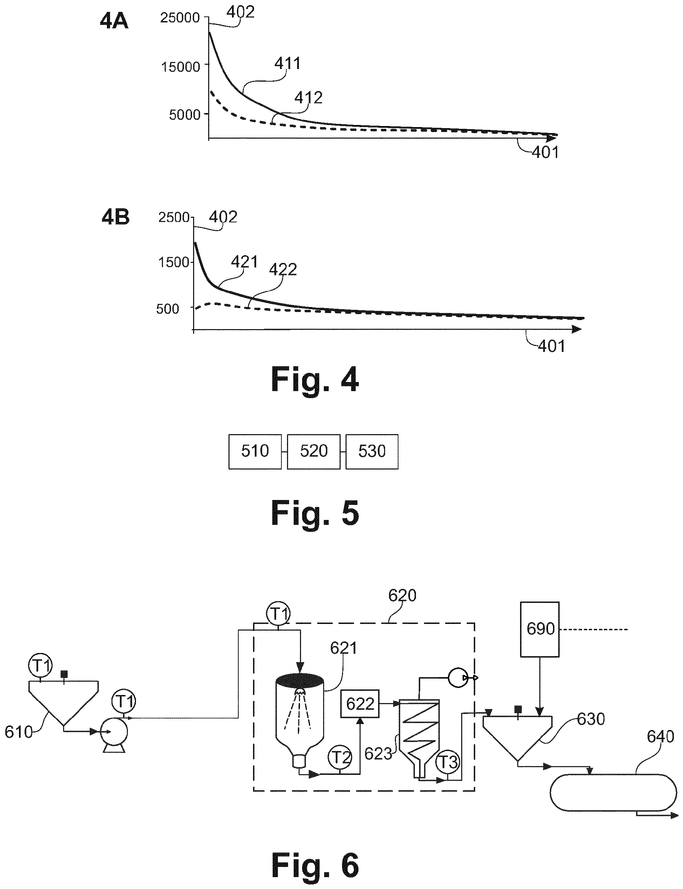

Figure 4, cet effet de dégradation est par exemple observé en soumettant le produit, à une température donnée, à des taux de cisaillement (401) croissants, puis à des taux de cisaillements décroissants. Un cycle de sollicitations qui mime celui subi par le produit lors d'une opération de stérilisation thermique en continu.

Figure 4A, l'essai est réalisé sur un même échantillon d'un produit correspondant au baume de la figure 3, à une température de 25°C. L'échantillon est d'abord soumis à des taux de cisaillement croissants (411) de 7 s$^{-1}$ à 150 s$^{-1}$, puis à des taux de cisaillements décroissants (412) de 150 s$^{-1}$ à 7 s$^{-1}$.

À l'issue de l'essai, la viscosité (402) du produit a chuté de 21 700 cP à 10 000 cP. La consistance du produit qui était celle d'un baume initialement se rapproche de celle d'une crème.

Figure 4B le même essai est réalisé, avec un autre échantillon du baume, cette fois à 50 °C. À l'issue de l'essai la viscosité (402) du produit n'est plus que de 500 cP à 50°C, soit très fluide, et de plus, le produit perd son caractère émulsif avec la séparation des phases grasse et aqueuse.

Cet effet est notamment constaté sur la non-remontée de la viscosité pour les faibles taux de cisaillement constaté sur la courbe « retour » (422) correspondant au taux de cisaillement décroissants en regard de la courbe « aller » (421).

Cet effet de dégradation de la viscosité du produit, voire de la dégradation pure et simple du produit par séparation des phases, lorsque celui-ci est soumis à un cycle de sollicitation thermomécanique est particulièrement sensible lorsque le produit est très visqueux.

À titre d'exemple, en pratiquant l'essai de la figure 4 sur des échantillons de la crème de la figure 3, les courbes aller et retour se superposent quelle que soit la température de l'essai comprise entre 25°C et 60°C.

Or, tous les procédés de stérilisation thermique en continu, soumettent le produit traité à des sollicitations thermomécaniques intenses de ce type, et ce d'autant plus que le débit de traitement est élevé.

De plus, dans certains cas, courants dans le cas des produits très visqueux, la dégradation par effet thermomécanique de l'émulsion est irréversible : il n'est plus possible, ou plus économique, de retrouver les qualités organoleptiques de l'émulsion, même en soumettant celle-ci à une forte agitation.

Les produits visqueux visés par l'invention comprennent un gélifiant, par exemple sous la forme d'un polymère ou d'un copolymère d'acide acrylique ou méthacrylique réticulé présent dans la phase continue de l'émulsion.

L'effet viscosant et stabilisant de ce polymère est obtenu par le déroulement des chaînes moléculaires dudit polymère, cet effet est obtenu par la neutralisation du polymère acide en ajoutant à la composition des éléments alcalins tels que de l'hydroxyde de sodium ou de la triéthanolamine.

Selon des variantes de réalisation, d'autres polymères ou copolymères acides présentant des propriétés viscosantes sont utilisables, ils sont introduits dans la pré-émulsion ou dans l'une de ses phases à l'état non neutralisé ou partiellement neutralisé.

Selon les formulations, un tel gélifiant est présent entre 0,3 % et 1 % de la composition en poids sans que cette fourchette ne soit limitative. Le gélifiant est par exemple additionné à la composition sous la forme d'une poudre.

À l'état non neutralisé, les chaînes moléculaires sont enroulées et serrées, de sorte que ledit gélifiant, bien que présent, n'a pas ou très peu d'effet sur la viscosité du produit.

Figure 5, une première étape de pré-émulsion (510) du procédé objet de l'invention, consiste à obtenir une pré-émulsion qui comprend la base émulsive du produit, c'est-à-dire la phase continue aqueuse selon un exemple de réalisation, et toutes ou partie des matières grasses composant la phase dispersée.

Préférentiellement, toutes les matières grasses sont introduites dans la pré-émulsion. Préférentiellement également, tous les principes actifs sont introduits dans la pré-émulsion, de même, si le produit comprend un agent de consistance ou épaississant celui-ci est également introduit dans l'émulsion. Le gélifiant est introduit dans la pré-émulsion à l'état non neutralisé.

La pré-émulsion est obtenue par mélange sous agitation. L'objectif étant d'obtenir une pré-émulsion grossière.

La phase grasse et la phase aqueuse sont d'abord préparées séparément.

Selon un exemple de réalisation l'obtention de la phase aqueuse, comprend une phase de dispersion, réalisée selon un premier palier de température entre 30°C et 40°C sous agitation vive, au cours de laquelle le gélifiant non neutralisé est dispersé dans ladite phase aqueuse. La phase de dispersion est suivie d'une phase d'hydratation du gélifiant dans cette même gamme de température sous agitation lente.

Parallèlement la phase grasse est préparée en mélangeant ses constituants à une température comprise entre 80°C et 90°C.

La température de la phase aqueuse est portée à une température comprise entre 80°C et 90°C, à la même température que la phase grasse.

Les deux phases, grasses et aqueuses ont alors mélangées sous agitation de sorte à obtenir une pré-émulsion grossière, laquelle se trouve à une température T1 comprise entre 80°C et 90°C.

Bien qu'elle en comprenne la grande majorité des ingrédients cette pré-émulsion est très différente du produit finalisé. Elle est grossière, ce qui permet une obtention plus rapide avec des temps de malaxage réduits, elle n'est pas stable dans le temps, et sa viscosité à la température T1 est très faible, inférieur à 500 cP, préférentiellement inférieure à 300 cP.

Toutefois, cette faible viscosité rend ladite pré-émulsion robuste vis-à-vis des sollicitations thermomécaniques subies pendant le cycle de stérilisation et permet de la faire circuler à haut débit dans l'installation.

La pré-émulsion ainsi obtenue à la température T1 est dirigée directement vers une étape de stérilisation (520).

La stérilisation est obtenue en portant la pré-émulsion à une température T2, typiquement comprise entre 140°C et 145°C pendant un temps t adapté pour obtenir un F0 de 15 à la température de stérilisation, suivie d'un refroidissement à la température T1.

Ainsi, le procédé objet de l'invention supprime l'étape de préchauffage des procédés de stérilisation appliqués aux produits dermo-cosmétiques. L'écart entre T1 et T2 est également réduit, ce qui permet un chauffage plus rapide, particulièrement dans un procédé mettant en oeuvre un chauffage indirect.

Selon des variantes de réalisation, la stérilisation est obtenue par un chauffage direct, par exemple par infusion, ou par un chauffage indirect en faisant passer la pré-émulsion par des échangeurs thermiques.

Ces modes de réalisation sont détaillés plus bas en regard des installations pour la mise en oeuvre du procédé objet de l'invention.

Au cours d'une étape de finalisation (530) la pré-émulsion stérile et à la température T1 est dirigée vers un malaxeur stérile également à la température T1, comprise entre 80 °C et 90 °C.

La pré-émulsion subit d'abord une agitation vive afin de l'affiner. Puis les éléments alcalins nécessaires à la neutralisation du gélifiant sont introduits dans la pré-émulsion sous agitation lente.

Ces opérations finalisent le produit qui, après refroidissement en milieu stérile, atteint la viscosité désirée.

Selon une variante de mise en oeuvre, l'étape de finalisation comprend l'introduction de composants, sous agitation lente, à une température inférieure à T1, par exemple 40°C. Cette étape permet d'introduire des ingrédients de la composition qui seraient susceptibles de perdre une partie de leurs propriétés lors de la stérilisation.

Mais d'une manière générale il est préférable d'introduire tous les ingrédients, sauf les ingrédients de neutralisation du gélifiant, lors de la première étape (510) de malaxage. Tous les ingrédients introduits lors de l'étape de finalisation (530) sont stériles et ont été stérilisés préalablement à leur introduction par toute méthode adaptée.

Après l'étape de finalisation le produit est stocké en cuve stérile en vue de son conditionnement.

Quel que soit le mode de réalisation du procédé objet de l'invention, le procédé de stérilisation est un procédé continu, c'est-à-dire que le débit du produit est conservé tout le long du procédé.

Dans un procédé de traitement continu, le débit de traitement est fixé par les moyens de pompage répartis dans l'installation et qui amènent le produit d'un poste de traitement à un autre. Le débit atteignable est limité, entre autres, par la pression de pompage.

Cette pression est notamment le résultat des pertes de charges dynamiques subies par le produit transitant dans les différents conduits et moyens de l'installation.

Ainsi, le débit maximum possible est limité notamment par la pression maximale qui peut être utilisée lors du pompage, cette pression maximale étant limitée par la technologie des pompes utilisées mais aussi par l'effet de cette pression sur la qualité et les propriétés du produit.

D'une manière générale moins le produit est visqueux et plus les débits atteignables sont élevés.

Figure 6, selon un exemple de réalisation, le procédé objet de l'invention met en oeuvre un procédé de stérilisation par infusion.

L'installation pour la mise en oeuvre du procédé comprend un poste de mélange et d'émulsion (610) pour obtenir la pré-émulsion, dans lequel la température finale de réalisation de la pré-émulsion est égale à une température T1 comprise entre 80°C et 90°C.

À cette fin le poste de mélange (610) comprend des moyens de chauffage pour en contrôler la température. Cette opération de mélange est réalisée en environnement non stérile.

Ainsi, en sortant du poste de mélange (610) à la température T1, la pré-émulsion est dirigée vers le poste (620) de stérilisation par infusion.

Le poste de stérilisation (620) comprend un poste de chauffage par infusion (621), un poste de maintien en température (622) et un poste de refroidissement rapide (623) dit « flash ».

Le chauffage par infusion est réalisé en pulvérisant en jet la pré-émulsion dans une enceinte remplie de vapeur d'eau purifiée, portée à la température désirée.

La surface d'échange du produit avec la vapeur étant très élevée, le chauffage de la pré-émulsion à la température de stérilisation T2 est quasiment instantané dans tout le volume injecté dans la chambre de stérilisation.

Au cours de cet échauffement, la pré-émulsion absorbe de l'eau correspondant à la condensation de la quantité de vapeur lui ayant transmis sa chaleur.

Le poste de maintien en température (622) permet de maintenir la pré-émulsion ainsi chauffé à la température atteinte lors du chauffage par infusion, afin d'obtenir le niveau de stérilité visé.

Ce poste de maintien, ou de chambrage, consiste en un conduit de longueur adaptée en fonction du débit et situé entre le poste de chauffage par infusion (621) et le poste de refroidissement (623). La longueur du conduit définit ainsi le temps de maintien pour un débit de traitement donné.

À l'issue de ce maintien, la pré-émulsion, à la température de stérilisation T2, entre dans le poste de refroidissement (623).

Dans ce poste de refroidissement, la pré-émulsion, chauffée à la température T2, est mise en communication avec une chambre mise sous vide.

Le produit aspiré dans cette chambre subit une détente brusque accompagnée d'une libération violente de vapeur.

La chaleur latente de vaporisation prélève de l'énergie thermique au produit et ainsi le refroidit, jusqu'à une température T3.

L'eau est récupérée sous forme de vapeur dans le haut du refroidisseur, alors que la pré-émulsion, débarrassée de l'eau absorbée lors du chauffage par infusion, est collecté et récupérée en bas du refroidisseur.

Le cycle thermodynamique est réglé de sorte que l'eau absorbée lors du chauffage par infusion du cycle de stérilisation est récupérée sous forme de vapeur lors du cycle de refroidissement flash. Ainsi, les températures T1 et T3 sont nécessairement proches, et T3 est comprise entre T1 et T1-5°C.

A l'issue de la stérilisation, la pré-émulsion, à la température T3 proche de la température T1, est dirigée vers un poste de malaxage (630) stérile, lui-même à la température T3 et comprenant des moyens pour en contrôler la température. Les autres composants de la formulation, en particulier les ingrédients alcalins pour neutraliser le gélifiant, sont alors ajoutés à la pré-émulsion à la température T3 après avoir eux-mêmes subi une étape (690) de stérilisation par tout procédé adapté.

Après refroidissement, le produit finalisé et stable est dirigé vers une cuve stérile (640) en vue de son conditionnement.

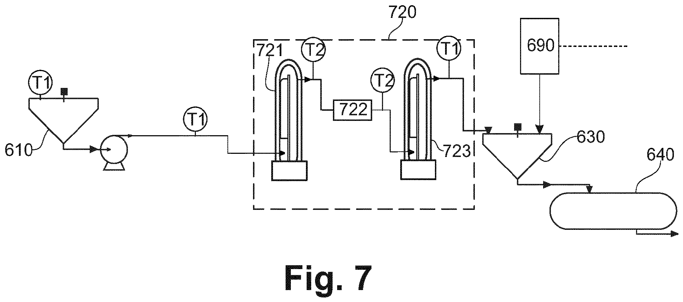

Au cours de l'opération de stérilisation par infusion la pré-émulsion absorbe de l'eau, provenant de la vapeur présente dans l'infuseur, eau qui est évacuée lors du refroidissement flash. Dans certaines circonstances ou pour certains produits cette absorption-résorption d'eau n'est pas désirée. L'installation représentée figure 7 résout ce problème.

Figure 7, selon un mode de réalisation, l'installation pour la mise en oeuvre du procédé objet de l'invention comprend un poste de stérilisation (720) par chauffage indirect, les autres postes de l'installation restant les mêmes que ceux de l'installation représentée figure 6.

Le poste de stérilisation (720) comprend un poste de chauffage (721) par chauffage indirect, préférentiellement au moyen d'un échangeur thermique à surface raclée.

Un tel échangeur thermique comprend un stator et un rotor pourvu de pâles. Le stator comprend une double paroi chauffée à la température désirée par une circulation fluide, notamment de la vapeur, de sorte que la paroi interne de l'échangeur se trouve à une température de consigne, en l'occurrence la température T2 de stérilisation de l'ordre de 140°C.

Selon un mode de réalisation, le poste de chauffage (721) comprend plusieurs échangeurs thermiques à paroi raclée en parallèle.

La pré-émulsion quitte l'échangeur thermique à la température T2, pour un poste de chambrage (722) où elle est maintenue à cette température pendant un temps t adapté pour obtenir un niveau de stérilité F0 supérieur ou égal à 15. À titre d'exemple t= 10 secondes pour T2=141 °C.

Le poste de chambrage est similaire dans sa conception à celui de l'installation de la figure 6.

En sortie du poste de chambrage (722) la pré-émulsion à la température T2, entre dans un poste de refroidissement (723) constitué lui aussi préférentiellement d'un ou plusieurs échangeurs thermiques à paroi raclée. Par refroidissement la pré-émulsion est portée à la température T1.

Elle quitte le poste de refroidissement (723) à la température T1 pour rejoindre le malaxeur stérile (630) à cette même température. La suite de la mise en oeuvre est identique à celle de l'installation de la figure 6.

La pré-émulsion étant fluide, sa viscosité ne change quasiment pas depuis la sortie du premier malaxeur (610) jusqu'à l'entrée dans le second malaxeur (630). Cette constance de la viscosité permet un meilleur contrôle du débit dans un tel procédé de traitement en continu, et d'utiliser des pressions de circulations réduites pour l'obtention d'un débit donné.

La description ci-avant et les exemples de réalisation, montrent que l'invention atteint le but visé, et permet par le contrôle de la viscosité du produit le long du procédé d'atteindre des débits de traitement élevés sans dégradation et sans perte de viscosité du produit.

À titre d'exemple, le procédé objet de l'invention mettant en oeuvre la même installation de stérilisation par infusion, et pour un même produit, permet d'atteindre un débit de 4m³/h là où la mise en oeuvre d'un procédé sans l'utilisation d'une pré-émulsion ne permet pas d'atteindre 2m³/h.

**Revendications**

1. Procédé continu pour la stérilisation d'un produit dermo-cosmétique dans lequel le débit est conservé le long des étapes i) à iii), le produit dermo-cosmétique comprenant une base émulsive comprenant au moins une phase grasse et une phase aqueuse ainsi qu'un gélifiant polymère acide, comprenant les étapes consistant à :

    i. émulsionner (510) la base émulsive, comprenant le gélifiant à l'état non neutralisé, à une température T1, de sorte à obtenir une pré-émulsion ;
    ii. stériliser (520) la pré-émulsion par un procédé thermique apte à obtenir un abattement d'au moins 15 Log du nombre de germes dans une unité de volume de la pré-émulsion traitée ;
    iii. finaliser (530) le produit dans un poste de malaxage stérile (630) à une température égale ou inférieure à T1, l'étape de finalisation comprenant la neutralisation du gélifiant de sorte à obtenir la viscosité finale et la stabilité du produit.

2. Procédé selon la revendication 1, dans lequel T1 est comprise entre 80°C et 90°C.

3. Procédé selon la revendication 1, dans lequel l'étape (ii) comprend les étapes consistant à :

iia. porter la pré-émulsion de la température T1 à la température T2 par infusion de vapeur;
ii.b. maintenir la pré-émulsion à la température T2 pendant un temps t de sorte à obtenir un abattement d'au moins 15 Log du nombre de germes dans une unité de volume de la pré-émulsion traitée ;
ii.c. refroidir la pré-émulsion à une température comprise entre T1 et T1-5°C par refroidissement flash.

4. Procédé selon la revendication 3, dans lequel T2 est compris entre 141°C et 145°C et t est compris entre 10 secondes et 4 secondes.

5. Procédé selon la revendication 3, dans lequel l'étape (i) consiste à obtenir une pré-émulsion grossière comprenant des tailles et une répartition des micelles non homogènes.

6. Procédé selon la revendication 1, dans lequel l'étape ii) comprend les étapes consistant à :

ii.x. porter la pré-émulsion de la température T1 à la température T2 par un chauffage indirect dans un échangeur de chaleur (721) ;
ii.y. maintenir la pré-émulsion à la température T2 dans l'échangeur de chaleur pendant un temps t de sorte à obtenir un abattement d'au moins 15 Log du nombre de germes dans une unité de volume de la pré-émulsion traitée ;
ii.z. refroidir la pré-émulsion à une température égale ou inférieure à T1 par un refroidissement indirect dans un échangeur de chaleur (723).

7. Procédé selon la revendication 6, dans lequel dans lequel l'étape i) consiste à obtenir une pré-émulsion grossière comprenant des tailles et une répartition des micelles non homogènes.

8. Installation pour la mise en oeuvre d'un procédé selon l'une quelconque des revendications 3 à 5, comprenant :

a. un premier poste de malaxage (610) comprenant des moyens de chauffage pour obtenir une pré-émulsion comprenant le gélifiant à l'état non neutralisé ;
b. un poste (620) de stérilisation par infusion comprenant un poste de chauffage par infusion (621) dans lequel la pré-émulsion est pulvérisée en jet dans une enceinte remplie de vapeur d'eau purifiée portée à la température de stérilisation, un poste de maintien en température (622) et un poste de refroidissement rapide (623) ;
c. un deuxième poste de malaxage stérile (630) à la sortie du poste de stérilisation et comprenant des moyens pour en contrôler la température et des moyens pour introduire dans ce deuxième poste de malaxage stérile les composants stériles pour la neutralisation du gélifiant ;

dans lequel la pré-émulsion, obtenue dans le premier poste de malaxage (610) à la température T1 est introduite directement dans le poste de stérilisation (620) par infusion à la température T1 sans passer par un moyen de préchauffage intermédiaire.

9. Installation pour la mise en oeuvre du procédé selon la revendication 6 ou la revendication 7, comprenant :

u. un premier poste de malaxage (610) comprenant des moyens de chauffage pour obtenir une pré-émulsion comprenant le gélifiant à l'état non neutralisé ;
v. un poste de chauffage indirect (721) de la pré-émulsion à la température T2 de stérilisation ;
w. un poste de maintien (722) de la pré-émulsion à la température T2 pendant un temps t de sorte à obtenir un abattement d'au moins 15 Log du nombre de germes dans une unité de volume de la pré-émulsion traitée;
x. un poste de refroidissement (723) de la pré-émulsion par refroidissement indirect ;
y. un deuxième poste de malaxage stérile (630) à la sortie du poste de stérilisation et comprenant des moyens pour en contrôler la température et des moyens pour introduire dans ce deuxième poste de malaxage stérile les composants stériles pour la neutralisation du gélifiant ;

dans lequel la pré-émulsion, obtenue dans le premier poste de malaxage (610) à la température T1 est introduite directement dans le poste de chauffage (721) à la température T1 sans passer par un moyen de préchauffage intermédiaire.

**Patentansprüche**

1. Kontinuierliches Verfahren zur Sterilisation eines dermokosmetischen Produkts, bei dem der Durchsatz entlang der Schritte i) bis iii) gehalten wird, wobei das dermokosmetische Produkt eine Emulsionsbasis, die mindestens eine Fettphase und eine Wasserphase umfasst, sowie ein saures polymeres Geliermittel umfasst, die folgenden Schritte umfassend, die darin bestehen:

i. die Emulsionsbasis, die das Geliermittel im nicht neutralisierten Zustand umfasst, bei einer Temperatur T1 zu emulgieren (510), um eine Voremulsion zu erhalten;

ii. die Voremulsion durch ein thermisches Verfahren, das geeignet ist, eine Reduzierung der Keimzahl in einer Volumeneinheit der behandelten Voremulsion von mindestens 15 Log zu erhalten, zu sterilisieren (520);

iii. das Produkt in einer sterilen Mischstation (630) bei einer Temperatur von gleich oder kleiner als T1 fertigzustellen (530), wobei der Schritt des Fertigstellens das Neutralisieren des Geliermittels umfasst, um die endgültige Viskosität und die Stabilität des Produkts zu erhalten.

2. Verfahren nach Anspruch 1, wobei T1 im Bereich zwischen 80 °C und 90 °C liegt.

3. Verfahren nach Anspruch 1, wobei Schritt (ii) die folgenden Schritte umfasst, die darin bestehen:

ii.a. die Voremulsion durch Dampfinfusion von der Temperatur T1 auf die Temperatur T2 zu bringen;

ii.b. die Voremulsion während einer Zeit t auf der Temperatur T2 zu halten, um eine Reduzierung der Keimzahl in einer Volumeneinheit der behandelten Voremulsion von mindestens 15 Log zu erhalten;

ii.c. die Voremulsion durch Blitzkühlung auf eine Temperatur im Bereich zwischen T1 und T1-5 °C zu kühlen.

4. Verfahren nach Anspruch 3, wobei T2 im Bereich zwischen 141 °C und 145 °C liegt und t im Bereich zwischen 10 Sekunden und 4 Sekunden liegt.

5. Verfahren nach Anspruch 3, wobei Schritt (i) darin besteht, eine grobe Voremulsion zu erhalten, die inhomogene Größen und eine inhomogene Verteilung der Mizellen umfasst.

6. Verfahren nach Anspruch 1, wobei Schritt ii) die folgenden Schritte umfasst, die darin bestehen:

ii.x. die Voremulsion durch ein indirektes Erhitzen in einem Wärmetauscher (721) von der Temperatur T1 auf die Temperatur T2 zu bringen;

ii.y. die Voremulsion während einer Zeit t auf der Temperatur T2 im Wärmetauscher zu halten, um eine Reduzierung der Keimzahl in einer Volumeneinheit der behandelten Voremulsion von mindestens 15 Log zu erhalten;

ii.z. die Voremulsion durch ein indirektes Kühlen in einem Wärmetauscher (723) auf eine Temperatur von gleich oder kleiner als T1 zu kühlen.

7. Verfahren nach Anspruch 6, wobei Schritt i) darin besteht, eine grobe Voremulsion zu erhalten, die inhomogene Größen und eine inhomogene Verteilung der Mizellen umfasst.

8. Anlage zur Umsetzung eines Verfahrens nach einem der Ansprüche 3 bis 5, umfassend:

a. eine erste Mischstation (610), die Heizmittel umfasst, zum Erhalten einer Voremulsion, die das Geliermittel im nicht neutralisierten Zustand umfasst;

b. eine Infusionssterilisationsstation (620), die eine Infusionsheizstation (621), in der die Voremulsion als Strahl in einer Kammer, die mit gereinigtem, auf die Sterilisationstemperatur gebrachten Wasserdampf gefüllt ist, pulverisiert wird, eine Temperaturhaltestation (622) und eine Schnellkühlstation (623) umfasst;

c. eine sterile zweite Mischstation (630) am Ausgang der Sterilisationsstation und Mittel zum Steuern ihrer Temperatur und Mittel zum Einführen der sterilen Komponenten in diese sterile zweite Mischstation zum Neutralisieren des Geliermittels umfassend;

wobei die Voremulsion, die in der ersten Mischstation (610) auf der Temperatur T1 erhalten wird, durch Infusion auf der Temperatur T1 direkt in die Sterilisationsstation (620) eingeführt wird, ohne ein zwischengeschaltetes Vorheizmittel zu durchlaufen.

9. Anlage zur Umsetzung des Verfahrens nach Anspruch 6 oder Anspruch 7, umfassend:

u. eine erste Mischstation (610), die Heizmittel umfasst, zum Erhalten einer Voremulsion, die das Geliermittel im nicht neutralisierten Zustand umfasst;

v. eine Station zum indirekten Erhitzen (721) der Voremulsion auf die Sterilisationstemperatur T2;

w. eine Station zum Halten (722) der Voremulsion während einer Zeit t auf der Temperatur T2, um eine Reduzierung der Keimzahl in einer Volumeneinheit der behandelten Voremulsion von mindestens 15 Log zu erhalten;

x. eine Station zum Kühlen (723) der Voremulsion durch indirekte Kühlung;

y. eine sterile zweite Mischstation (630) am Ausgang der Sterilisationsstation und Mittel zum Steuern ihrer Temperatur und Mittel zum Einführen der sterilen Komponenten in diese sterile zweite Mischstation zum Neutralisieren des Geliermittels umfassend;

wobei die Voremulsion, die in der ersten Mischstation (610) auf der Temperatur T1 erhalten wird, auf der Temperatur T1 direkt in die Heizstation (721) eingeführt wird, ohne ein zwischengeschaltetes Vorheizmittel zu durchlaufen.

**Claims**

1. Continuous method for the sterilisation of a dermo-cosmetic product wherein the flow rate is maintained during steps i) to iii), the dermo-cosmetic product comprising an emulsive base comprising at least a fatty phase and an aqueous phase, as well as an acid polymer gelling agent, comprising the steps of:

   i. emulsifying (510) the emulsive base, comprising the gelling agent in the non-neutralised state, at a temperature T1, so as to obtain a pre-emulsion;
   ii. sterilising (520) the pre-emulsion by means of a thermal process capable of achieving a reduction of at least 15 Log of the number of germs in a unit of volume of the treated pre-emulsion;
   iii. finalising (530) the product in a sterile mixing station (630) at a temperature equal to or less than T1, the finalising step comprising the neutralisation of the gelling agent so as to obtain the final viscosity and stability of the product.

2. Method according to claim 1, wherein T1 is between 80°C and 90°C.

3. Method according to claim 1, wherein step (ii) comprises the steps of:

   ii.a. bringing the pre-emulsion from the temperature T1 to temperature T2 by steam infusion;
   ii.b. holding the pre-emulsion at the temperature T2 for a time t so as to obtain a reduction of at least 15 Log of the number of germs in a unit of volume of the treated pre-emulsion;
   ii.c. cooling the pre-emulsion to a temperature between T1 and T1-5°C by flash cooling.

4. Method according to claim 3, wherein T2 is between 141°C and 145°C and t is between 10 seconds and 4 seconds.

5. Method according to claim 3, wherein step (i) consists of obtaining a coarse pre-emulsion comprising nonhomogeneous micelle sizes and distribution.

6. Method according to claim 1, wherein step ii) comprises the steps of:

   ii.x. bringing the pre-emulsion from the temperature T1 to temperature T2 by indirect heating in a heat exchanger (721) ;
   ii.y. holding the pre-emulsion at temperature T2 in the heat exchanger for a time t so as to obtain a reduction of at least 15 Log of the number of germs in a unit of volume of the treated pre-emulsion;
   ii.z. cooling the pre-emulsion to a temperature equal to or less than T1 by indirect cooling in a heat exchanger (723).

7. Method according to claim 6, wherein step i) consists of obtaining a coarse pre-emulsion comprising nonhomogeneous micelle sizes and distribution.

8. Installation for implementing a method according to any one of claims 3 to 5, comprising:

   a. a first mixing station (610) comprising heating means for obtaining a pre-emulsion comprising the gelling agent in the non-neutralised state;
   b. an infusion sterilisation station (620) comprising an infusion heating station (621) wherein the pre-emulsion is jet sprayed into an enclosure filled with purified water steam at the sterilisation temperature, a temperature-holding station (622) and a rapid cooling station (623);
   c. a sterile second mixing station (630) at the outlet of the sterilisation station and comprising means for controlling its temperature and means for introducing into this sterile second mixing station the sterile ingredients for neutralising the gelling agent;

   wherein the pre-emulsion, obtained in the first mixing station (610) at temperature T1 is introduced directly into the infusion sterilisation station (620) at temperature T1 without passing via an intermediate preheating means.

9. Installation for implementing the method according to claim 6 or claim 7, comprising:

   u. a first mixing station (610) comprising heating means for obtaining a pre-emulsion comprising the gelling agent in the non-neutralised state;
   v. a station (721) for indirect heating of the pre-emulsion to the sterilisation temperature T2;
   w. a station (722) for holding the pre-emulsion at temperature T2 for a time t so as to obtain a reduction of at least 15 Log of the number of germs in a unit of volume of the treated pre-emulsion;
   x. a station (723) for cooling the pre-emulsion by indirect cooling;
   y. a sterile second mixing station (630) at the outlet of the sterilisation station and comprising means for controlling its temperature and means for introducing into this sterile second mixing station the sterile ingredients for neutralising the gelling agent;

wherein the pre-emulsion, obtained in the first mixing station (610) at temperature T1 is introduced directly into the heating station (721) at temperature T1 without passing via an intermediate preheating means.

**1A**       **1B**

# Fig. 1

**2A**    212    211      **2B**    222    221

210      220

# Fig. 2

# Fig. 3

**4A**

**4B**

# Fig. 4

| 510 | 520 | 530 |

# Fig. 5

# Fig. 6

# Fig. 7

**RÉFÉRENCES CITÉES DANS LA DESCRIPTION**

**Documents brevets cités dans la description**

- WO 2013007755 A
- WO 2007148022 A
- EP 1588697 A